# EUROPEAN PATENT SPECIFICATION

(11) **EP 3 618 900 B1**
(45) Date of publication and mention of the grant of the patent: **15.10.2025**
(21) Application number: 18719919.5
(22) Date of filing: 02.05.2018
(51) Int. Cl.: A61M 5/315

(54) **INTEGRATING ELECTRONICS INTO THE STOPPER OF A CARTRIDGE**
INTEGRATION VON ELEKTRONIK IN EINEM STOPFEN EINER KARTUSCHE
INTÉGRATION D'ÉLECTRONIQUE DANS LE BOUCHON D'UNE CARTOUCHE

(30) Priority: 05.05.2017 EP 17305515
(43) Date of publication of application: 11.03.2020
(73) Proprietor: Sanofi, 75008 Paris (FR)
(72) Inventor: SCHABBACH, Michael, 65926 Frankfurt am Main (DE); ALLERDINGS, Alexander, 65926 Frankfurt am Main (DE); DETTE, Christoph, 65926 Frankfurt am Main (DE); OTTEN, Martin, 65926 Frankfurt am Main (DE); POMMEREAU, Christian, 65926 Frankfurt am Main (DE); HAMMEN, Dietmar, 65926 Frankfurt (DE); JANSEN, Paul Edward, Boston, Massachusetts 02115 (US); BRUEGGEMANN, Ulrich, Bridgewater, New Jersey 08807 (US); ILNICKI, Iwo, 65926 Frankfurt am Main (DE)
(74) Representative: Weilnau, Carsten
(86) International application number: PCT/EP2018/061110
(87) International publication number: WO 2018/202660

(56) References cited:
- EP-A1- 2 190 506
- WO-A1-2009/032399
- WO-A1-2016/036574
- WO-A2-2017/070391
- US-A1- 2007 219 507
- US-A1- 2011 213 311
- US-A1- 2015 196 715
- US-A1- 2015 217 059

## Description

This description relates to an apparatus for a medical device configured to eject a medicament, and more particularly to a stopper for advancing a medicament in a cartridge of an injection device.

A variety of diseases exist that require treatment by injection of a medicament. Such injection can be performed using injection devices, which are applied either by medical personnel or by patients themselves. As an example, type-1 and type-2 diabetes can be treated by patients themselves by injection of drug doses, for example once or several times per day. For instance, a pre-filled disposable drug pen or autoinjector can be used as an injection device. Alternatively, a re-usable pen or autoinjector may be used. A re-usable pen or autoinjector allows replacement of an empty medicament cartridge by a new one. Either type of pen or autoinjector may come with a set of one-way needles that are replaced before each use.

An example embodiment is a stopper configured to be disposed within a medical cartridge, the stopper including a shell defining a cavity and an exterior surface sized and shaped to fit inside the medical cartridge, and an insert configured to be inserted into the cavity. The insert includes an electronic device having a sensor configured to generate a sensing signal. The shell is configured to be advanced into the medical cartridge by a plunger to apply pressure to a substance contained in the medical cartridge. The shell is configured to pass the sensing signal through the material of the shell, and the sensor is configured to be responsive to the position of the stopper in the medical cartridge.

In some embodiments, the insert includes a cap member configured to enclose the cavity when the insert is inserted into the cavity, and the cap member is configured to receive a driving force from the plunger to advance the stopper in the medical cartridge.

In some embodiments, the insert is configured to be secured to the shell using one or more of the following: a snap feature, glue, welding, ultrasonic welding, or thermal welding.

In some embodiments, the shell is constructed from a material that can be heat sterilized.

In some embodiments, the insert is a distal end of the plunger including mating features and the cavity includes corresponding mating features configured to secure the insert to the shell when the distal end of the plunger is inserted into the cavity of the shell of the stopper. The cavity includes guiding features arranged to oriented and position the electronic device in the shell.

In some embodiments, the shell is a rigid shell made from a substantially rigid material. In some embodiments, the stopper includes a sealing member disposed around the exterior surface and arranged to form a seal between the exterior surface and an inner surface of the medical cartridge when the stopper is disposed within the medical cartridge

In some embodiments, the shell is a soft shell made from a substantially pliable material. In some embodiments, the exterior surface of the soft shell defines a sealing protrusion extending radially from exterior surface and arranged to form a seal between the exterior surface and an inner surface of the medical cartridge when the stopper is disposed within the medical cartridge

In some embodiments, the sensing signal is an electromagnetic signal or an acoustic signal. In some embodiments, the electronic device is configured to communicate with an external electronic device. In some embodiments, the electronic device includes a wireless transceiver configured to communicate with the external electronic device. In some embodiments, the wireless transceiver is configured to transmit data acquired by the sensor to the external electronic device. In some embodiments, the electronic device is configured to be activated by a force applied to the stopper.

In some embodiments, the electronic device includes a temperature sensing element configured to activate the electronic device upon sensing a change in temperature or upon reaching temperature threshold.

In some embodiments, the electronic device includes an energy source. In some embodiments, the energy source includes a wireless charging module having a capacitive electronic circuit. In some embodiments, the energy source includes a piezoelectric element configured to generate electric energy from a force applied to the stopper. In some embodiments, the energy source is a thermoelectric element configured to generate electric energy from heat energy absorbed by the stopper.

Another example embodiment is a medical cartridge having a cartridge housing and the stopper described above, where the shell is configured to be inserted into the cartridge housing prior to the cartridge housing and shell undergoing a heat sterilization procedure, and the insert is configured to be inserted into the stopper after the heat sterilization procedure.

In some embodiments, the medical cartridge is a syringe, and the cartridge housing is a housing of the syringe, and the insert is a rod of a plunger of the syringe configured to be inserted into the shell of the stopper after the syringe undergoes the heat sterilization procedure.

Yet another example embodiment is a method of manufacturing a medical cartridge. The method includes inserting a shell into a housing of the medical cartridge, the stopper sealing an open end of the housing, the shell defining a cavity accessible outside the housing and configured to pass a sensing signal therethrough, heat sterilizing the shell and housing, and after the heat sterilizing, placing an insert into the cavity of the shell, the insert including an electronic device including a sensor configured to generate the sensing signal. The shell and insert together form a stopper configured to be advanced into the medical cartridge by a plunger to apply pressure to a substance contained in the medical cartridge, and the sensor is configured to be responsive to the position of the stopper in the medical cartridge.

### DESCRIPTION OF DRAWINGS

FIG. 1 is an exploded view of an injection device.
FIG. 2A is a cross sectional view of a stopper configured to be disposed in an injection device, the stopper having a rigid shell and a soft insertable core with electronic devices embedded in the core.
FIG. 2B is a top view of the stopper of FIG. 2A.
FIG. 2C is a cross-sectional view of a stopper disposed within a cartridge.
FIG. 3A is a cross sectional view of a stopper configured to be disposed in an injection device, the stopper having a soft shell and a rigid insertable core with electronic devices embedded in the core.
FIG. 3B is a top view of the stopper of FIG. 3A.
FIG. 4 is a cross-sectional view of a stopper configured to be disposed in an injection device, the stopper having a rigid shell and a soft insertable core with electronic devices embedded in the core and having a rigid cover.
FIG. 5 is a cross-sectional view of a stopper configured to be disposed in an injection device, the stopper having a soft shell and a rigid insertable core with electronic devices embedded in the core.
FIG. 6 is a cross-sectional view of a stopper disposed within a cartridge.
FIG. 7A is cross-sectional view of a shell of a stopper disposed within a cartridge and an insert being coupled to the shell.
FIG. 7B is cross-sectional view the stopper of FIG. 7A containing the insert before being driven by a plunger.
FIG. 7C is cross-sectional view the stopper of FIG. 7B during a sensing operating during or after being driven into the cartridge by the plunger
FIGS. 8A and 8B are cross-sectional views of a disposable syringe having electronics embedded into a threaded distal end of the plunger.
FIG. 9 is a flowchart depicting a method of assembling and sterilizing a cartridge with a stopper before coupling electronic elements into the stopper.
FIG. 10 is cross-sectional view of a stopper disposed within a cartridge and communicating with a wireless system.
FIGS. 11A and 11B are cross-sectional views of a stopper disposed within a cartridge and powered by a piezoelectric system.
FIGS. 12A and 12B are cross-sectional views of a stopper disposed within a cartridge and Peltier thermoelectric device.
FIGS. 13A and 13B are cross-sectional views of a shell of a stopper disposed within a cartridge and an insert being clipped into a shell.

### DETAILED DESCRIPTION

Some cartridge-based injection and medical syringe systems can be difficult to sterilize prior to use if they include electronics in the replaceable portions of the device (e.g., a cartridge stopper or a cartridge housing). The electronic assembly may contain temperature sensitive materials (e.g. any polymers) which may get degraded after applying heat. Further, batteries can lose performance at high temperatures. In some examples, the cartridge stopper (sometimes referred to as a bung) can be sterilized before the electronics are added. For example, a stopper made of a rigid material can be sealed against the cartridge wall with soft 0-ring seals, and an insertable electronic device that is separated from the stopper can be used so that it can be assembled into the stopper or cartridge after the heat sterilization step (and, therefore, also without impacting the sterilized liquid inside the cartridge). Some examples also enable adding electronic components (e.g. RFID, sensors) to previously a heat-sterilized plunger of a disposable or reusable drug cartridge.

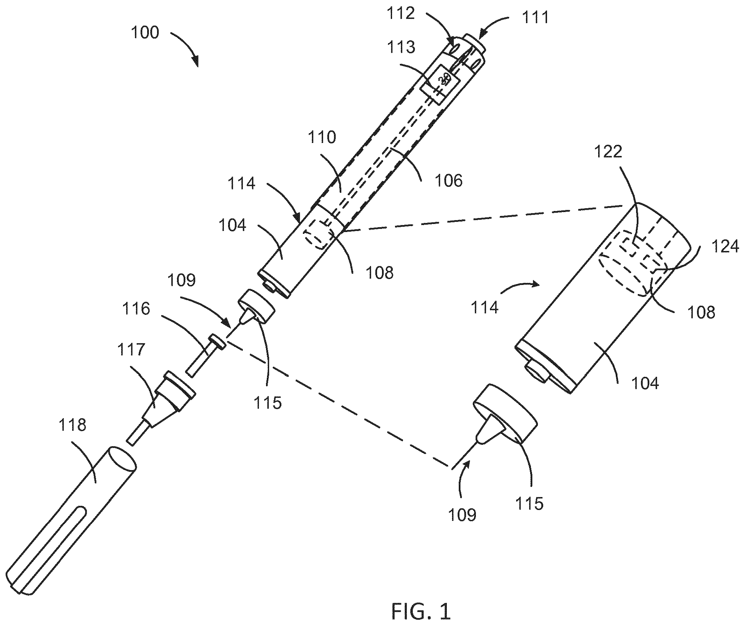

FIG. 1 is an exploded view of an injection device 100, which may be a disposable or reusable injection device. The injection device 100 includes a housing 110 and a having therein a cartridge 114 with cartridge housing 104, to which a needle assembly 115 can be affixed. The needle 109 of needle assembly 115 is protected by an inner needle cap 116 and an outer needle cap 117, which in turn can be covered by cap 118. A medicament or drug dose to be ejected from the injection device 100 is selected by turning a dosage knob 112, and the selected dose is displayed via a dosage window or display 113. The display can include a digital display, for example. The dosage display 113 relates to the section of the injection device through or on which the selected dosage is visible.

As described further below, the injection device 100 may include one or more electronic components 122, 124, some of which may be included in the stopper 108, for example.

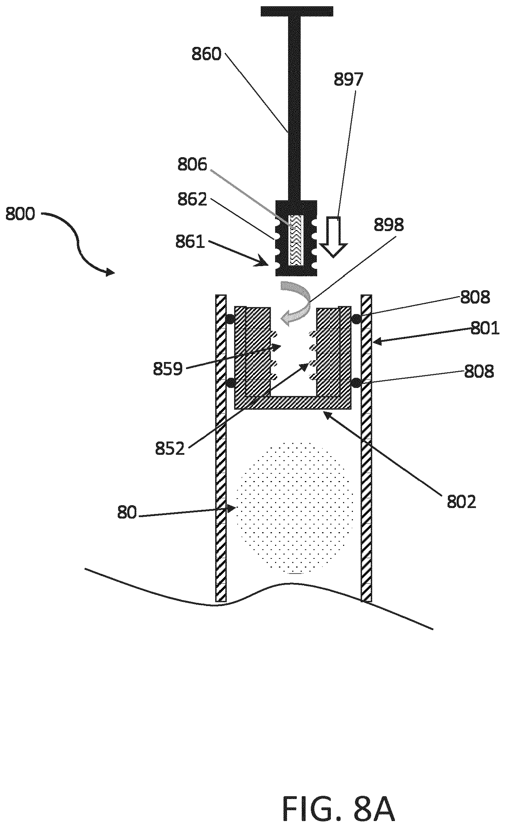

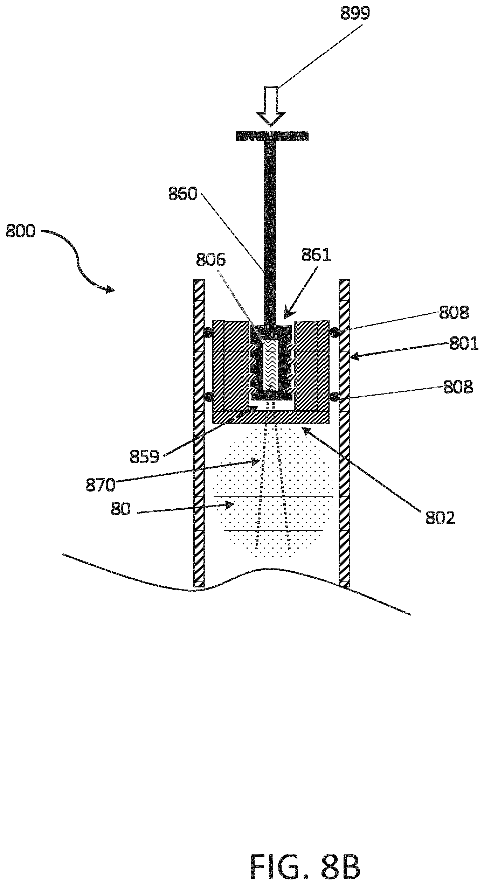

Turning the dosage knob 112 causes a mechanical click sound to provide acoustical feedback to a user. The numbers displayed in the dosage display 113 are printed on a sleeve that is contained in the housing 110 and mechanically interacts with a plunger in the cartridge 114. When the needle 109 is stuck into a skin portion of a patient, and then the injection button 111 is pushed, the drug dose displayed in the display 113 will be ejected from the injection device 100. During an injection, a drive mechanism 106, which is shown as an outline of a plunger arm, drives a stopper 108 into the cartridge to expel the drug. The stopper is an important element of the container closure system because it is a barrier for fluids leaking in and out; a gas barrier (e.g. oxygen), and prevents evaporation of H2O and other fluids. In some embodiments, the seal function is provided by elastic sealing elements (808 of FIG. 8A or Fig. 8B) in contact with the container walls, but still allows the stopper to glide. When the needle 109 of injection device 100 remains for a certain time in the skin portion after the injection button 111 is pushed, a high percentage of the dose is actually injected into the patient's body.

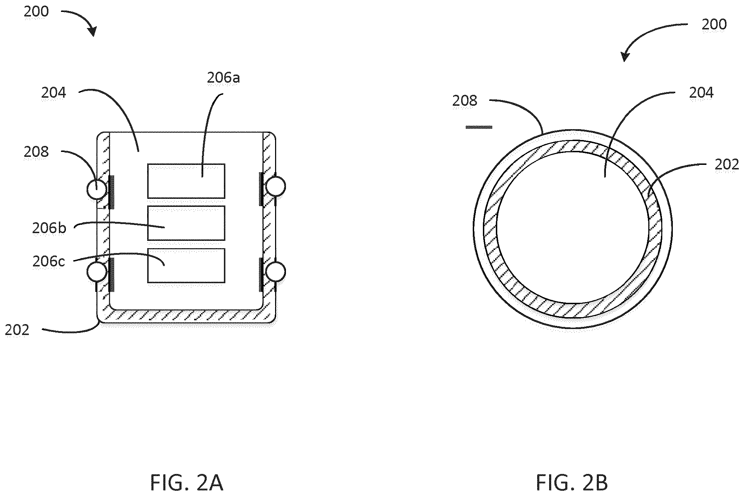

FIG. 2A is a cross sectional view of an embodiment of a stopper 200 configured to be disposed in an injection device (e.g., the injection device 100). The stopper 200 includes a rigid or substantially rigid shell 202 and a core 204 containing electronic devices 206a, 206b, and 206c, which, in some embodiments, are embedded into the material of the core. The core 204 is configured to be inserted into the shell 202 after manufacturing. The core 204 may comprise or form an insert configured for insertion into the cavity of the shell 202 and comprising an electronic device having a sensor configured to generate a sensing signal. For example, the core 204 may be inserted into the shell 202 after the shell is disposed into a cartridge of the injection device (e.g., cartridge 114 of FIG. 1). The shell 202 includes sealing elements 208 (e.g. an o-ring) that are arranged to provide a sealing interface with an inner surface of the cartridge 114 when the shell 202 is inserted into the cartridge 114. The sealing element 208 may form a sealing member disposed around the exterior surface of the shell 202. The sealing element s08 may define a sealing protrusion extending radially from the exterior surface of the shell 202.

The materials selected for the shell 202 and the core 204 are selected based on their hardness, elasticity, and their heat resistive or insulating properties. In some embodiments, the shell 202 and the core 204 are made of polymer materials with varying elastic properties. In some embodiments, heat resistive coatings may also be applied to the exterior of the core 204 or the shell 202 to increase heat resistance, such as, for example, a Teflon coating (e.g., polytetrafluoroethylene). In some cases, the shell 202 could be made of material which is selected to be compatible with the medicament e.g. PP, PE, POC, POP, PTFE or butyl rubber at the surface 203 which is in contact with the medicament. In some embodiments, the materials selected for the shell 202 and the core 204 are also selected for their ability to allow a sensor signal to pass through, for example, nonmetal material like polymers or ceramics or very thin metal (e.g., <0.1mm in thickness).

The embedded electronic devices 206a, 206b, and 206c may include, for example, a sensor, an energy source, a microcontroller, or a wireless transceiver. The embedded electronics 206a, 206b, 206c are representative only. There can be any number of embedded electronics. The sensor may be, in some embodiments, a sensor/transceiver device such as, for example, a piezoelectric device, an acoustic sensor, or an electromagnetic sensor. The sensor/transceiver may transmit a signal, such as, for example, an ultrasonic, acoustic, light, or other signal through the stopper 200 and measure a response which may, in some embodiments, be used to determine the position of the stopper 200 in a cartridge 114 or if an injection of the syringe has occurred. The position of the stopper 200 before and after an injection corresponds to a change in volume of medicament remaining in the cartridge 114, which can indicate the volume or dose of the medicament expelled from the cartridge 114 into the needle 109. In some embodiments, the response received by the sensor is provided to a controller (e.g. an embedded or an external microcontroller) which may receive the response and calculate a state of the cartridge 114. The state of the cartridge 114 may correspond to a fill level of medicament in the cartridge 114,a position of the stopper 200, or volume of a dose.

In some embodiments, the energy source can be a battery, an energy harvesting device generating energy by any energy harvesting technologies which may load a capacitor for temporary energy storage, or be solar powered. The wireless transceiver may communicate with an external electronic device as well as with the sensor and energy source. The external electronic device, which may be a controller, may communicate data received from the sensor to an external database. The communication with the controller can be one way or bidirectional. Data transferred from the sensor device to an external data base can contain information which is related to the identity of the device e.g. a unique number, calibration data, production lot information, device material information, data related to storage time and production time, and information related to the sensor measurement (e.g., time of measurement, sensor measurement results like temperature, distances, light signals, acoustic signals). In some embodiments, data coming from the external data base device to the controller contains information regarding "wake up" signals, triggers to measure, time information, or calibration data. The wireless transceiver may communicate using any known wireless communication technique including, for example Bluetooth, NFC, or radio frequencies.

In some embodiments, the shell 202 and the core 204 are manufactured separately and the core 204 is inserted into the shell 202 in a subsequent assembly step. For example, the core 204 may be formed upon filling a mold with a substantially soft material (e.g. by pouring, injecting, etc. the substantially soft material) and embedded electronic devices 206a, 206b, and 206c before the substantially soft material sets (e.g. cures, solidifies, etc.). In another embodiment, the substantially soft material is introduced surrounding the electronic devices 206a, 206b, and 206c and shaped (e.g. cut, poured into a mold, etc.) to form the core 204. The stopper 200 can include a sealing element 208 arranged around the periphery of the shell 202 to provide a sealing interface with the cartridge 114 upon the stopper's 200 introduction into the cartridge 114.

FIG. 2B is a top view of the stopper 200 of FIG. 2A. Shell 202 surrounds core 204 and interfaces with sealing element 208, which forms a sealing interface with the cartridge 114 upon the stopper's 200 introduction into the cartridge 114. The sealing interface may form at least part of a sterile barrier within the cartridge 114, which is required to preserve the sterility of the medicament to be delivered by the injection device.

The shell 202, in some embodiments, is constructed from of a rigid material such as metal, polymer (e.g., COC, PA, PP, PE, POM, PS, ABS, COP, etc.), glass or ceramics. In some embodiments, the electronic devices (or electronic assembly) 206a, 206b, 206c includes one or more of the following: a sensor, a power source (e.g. battery), a controller, a wireless communication module (e.g. Bluetooth, NFC, Bluetooth LE, any RF, IrDA, Acoustic module, memory, on-off switch, thermo-sensing element, pressure sensor etc. In some embodiments, the sealing members 208 are made from a first material and the shell 202 is made from a second material of lower compressibility compared to the first member's material, and where the shell 202 is formed as a receptacle to house the core 204. In some embodiments, the electronic devices 206a, 206b, 206c and the core 204 and are connected to the shell 202 using snap features, glue, welding, US welding, thermal welding or other means. In some embodiments, the core 204 is removably coupled to the shell 202. In some embodiments, the sealing member 208 is made of a natural rubber or any biocompatible composition of rubber. In some embodiments, the core 204 includes an on-off switch configured to trigger the electronics devices 206a, 206b, 206c by any suitable impact on the stopper 200 (e.g. force from a drive mechanism 106).

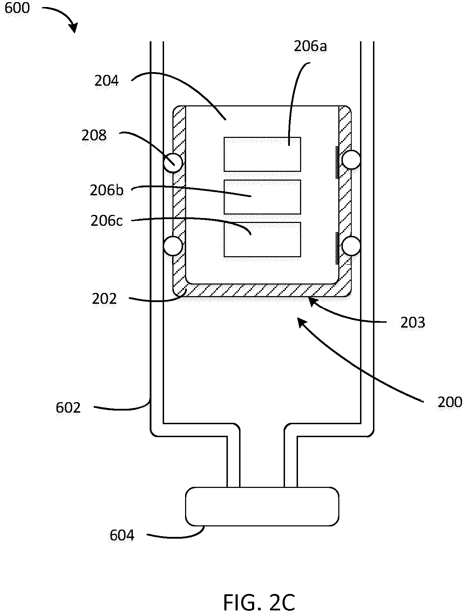

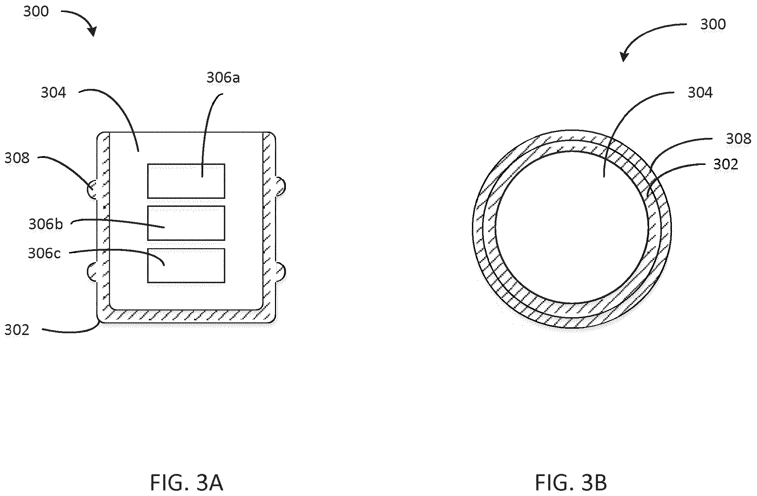

FIG. 2C is a cross-sectional view of a stopper 200 disposed within a cartridge 600. The various features of the stopper 200 shown are described above with respect to FIG. 3A and 3B. The stopper 200 may be replaced by any stopper of the following figures, which would interface with the cartridge 600 in much the same fashion as will be described here for stopper 300. The cartridge 600 includes a housing 602 which interfaces with the sealing element 208 of the stopper 200 to seal an open end of the cartridge 600. In some embodiments, a medicament is disposed in the space between the cap 604 of the cartridge 600 and the shell 202 of the stopper 200. In some embodiments, the embedded electronics 206a, 206b, and 206c, include a sensor configured to send and receive a signal to sense a position of the stopper 200 in the cartridge 600.

In an example, a transmitter (e.g., one of the electronics devices 206a, 206b, and 206c) transmits an acoustic wave at a first time *t*₁. The first time *t*₁ (e.g., the transmission time of the acoustic wave) may be provided to an external device. The acoustic wave propagates from the transmitter in the stopper 200 toward the distal end of the cartridge 600 and is reflected off of (e.g., bounces off of) a surface of the cartridge 600 or a reflector (not shown). A reflection of the acoustic wave (e.g., a reflected wave) propagates from the distal end of the cartridge 600 (i.e., the end having the cap 604) toward a sensor in the stopper 200. The reflected wave is received at a second time *t*₂. The speed of the acoustic wave is a known speed of sound in the medicament in the cartridge. The elapsed time between transmission and receiving of the acoustic wave is *t*₂ *- t*₁*.* The elapsed time is multiplied by the speed of sound to determine the distance traveled by the wave from the transmitter, to the distal end of the cartridge 600, back to the sensor. The distance traveled is divided by two to determine the distance between the stopper 200 and the distal end of the cartridge 600. The volume of medicament in the cartridge 600 (e.g., the volume of medicament enclosed in the cartridge 600 between the stopper 200 and the proximal end) is determined by multiplying the determined distance by the cross-sectional area of the cartridge 600. The difference in the determined volume of medicament in the cartridge 600 before and after dosing corresponds to the dose administered to the patient. FIG. 3A and 3B illustrate a stopper 300 configured to be disposed in an injection device 100, the stopper 300 having a soft shell 302 and a rigid core 304 with electronic devices 306a, 306b, 306c embedded in the core 304. In some embodiments, the rigid core 304 has the advantage to protect the electronics devices 306a, 306b, 306c from being deformed by the push of the plunger. This ensures reliable sensor signals. Another advantage is the rigid core 304 leads to better injection dose accuracy because of less elastic effects from the stopper 300 during injection. This also helps to shorten the holding time during injection. Soft in this context of the soft shell 302 means an visco-elastic material, for example, natural rubber, thermoplastic elastomers (TPE), butyl rubber, neoprene, fluroelastomers, silicone rubber etc., which are characterized by a low e-module (high elastic deformation and low remaining deformation und stress). The stopper 300 includes a substantially soft shell 302 and a substantially rigid core 304. In some embodiments, the shell 302 and the core 304 are substantially inseparable after assembly. For example, the core 304 may be inserted into the shell 302 and secured using an adhesive to bond the core 304 and the shell 302 together. An adhesive bond can be used between any of the core and shell/insert embodiments described herein. The core 304 contains embedded electronic devices 306a, 306b, and 306c. The core 304 may comprise or form an insert configured for insertion into the cavity of the shell 302 and comprising an electronic device having a sensor configured to generate a sensing signal. In one embodiment, a substantially rigid material may be molded around electronic devices 306a, 306b, and 306c while it is in an uncured, softened state and may solidify to form the rigid core 304. In another embodiment, the electronic devices306a, 306b, 306c may be introduced into the substantially rigid material while it is in an uncured, softened state before the substantially rigid material solidifies to form the rigid core 304. Integrated sealing element 308 forms a sealing interface (i.e., a gas-impermeable barrier) between the stopper and an interior wall of the cartridge 114 upon inserting the stopper 300 the cartridge 114. The sealing interface may form at least part of a sterile barrier within the cartridge 114 which is required to preserve the sterility of the medicament to be delivered by the injection device 100. The sealing element 308 may form a sealing member disposed around the exterior surface of the shell 302. The sealing element 308 may define a sealing protrusion extending radially from the exterior surface of the shell 302. The sealing element may form a seal between the exterior surface of the shell 302 and an inner surface of the medical cartridge when the stopper is disposed within the medical cartridge.

The various materials from which the shell 302 and the core 304 could be produced as well as the various electronic devices that may be embedded in the core are described above with respect to FIG. 2A. Additionally, the material for the shell 302 may be medical grade with the shell 302 being configured to come in contact with the medicament.

The shell of FIG. 2A, 2B, 3A, and 3B may be sterilized, for example, by using a heat sterilization process prior to the insertion of the core. In an example heat sterilization process, the shell would be sterilized at a temperature of approximately 120 degrees Celsius for between approximately twenty and thirty minutes, after which the core and associated electronics would be inserted into the shell.

The core 304, in some embodiments, is constructed from of a rigid material such as metal, polymer (e.g., COC, PA, PP, PE, POM, PS, ABS, COP, etc.), glass or ceramics. In some embodiments, the electronic devices (or electronic assembly) 306a, 306b, 306c includes one or more of the following: a sensor, a power source (e.g. battery), a controller, a wireless communication module (e.g. Bluetooth, NFC, Bluetooth LE, any RF, IrDA, Acoustic module, memory, on-off switch, thermo-sensing element, pressure sensor etc. In some embodiments, the sealing members 308 are made from a first material and the shell 302 is made from a second material of lower compressibility compared to the first member's material, and where the shell 302 is formed as a receptacle to house the core 304. In some embodiments, the electronic devices 306a, 306b, 306c and the core 304 and are connected to the shell 302 using snap features, glue, welding, US welding, thermal welding or other means. In some embodiments, the core 304 is removably coupled to the shell 302. In some embodiments, the sealing member 308 is made of a natural rubber or any biocompatible composition of rubber. In some embodiments, the core 304 includes an on-off switch configured to trigger the electronics devices 306a, 306b, 306c by any suitable impact on the stopper 300 (e.g. force from a drive mechanism 106).

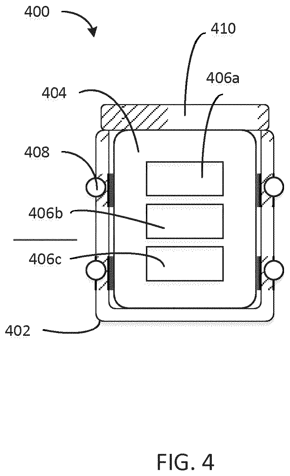

FIG. 4 is a cross-sectional view of a stopper 400 configured to be disposed in an injection device 100, the stopper 400 having a rigid shell 402 and a soft insertable core 404 with electronic devices 406a, 406b, 406c embedded in the core 404 and having a cover 410. In this embodiment, the shell 402 and the core 404 are manufactured separately and the core 404 is inserted into the shell 402 in a subsequent assembly step. For example, the core 404 may be formed upon filling a mold with a substantially soft material (e.g. by pouring, injecting, etc. the substantially soft material) and embedded electronic devices 406a, 406b, and 406c before the substantially soft material sets (e.g. cures, solidifies, etc.). In another embodiment, the substantially soft material is introduced surrounding the electronic devices 406a, 406b, and 406c and shaped (e.g. cut, poured into a mold, etc.) to form the core 404. The stopper 400 includes a sealing element 408 arranged around the periphery of the shell 402 to provide a sealing interface with the cartridge 114 upon the stopper's 400 introduction into the cartridge 114. Thus, in one embodiment there is a cover for the core and in another embodiment the need for a cover is eliminated, based on the particular configuration of the materials.

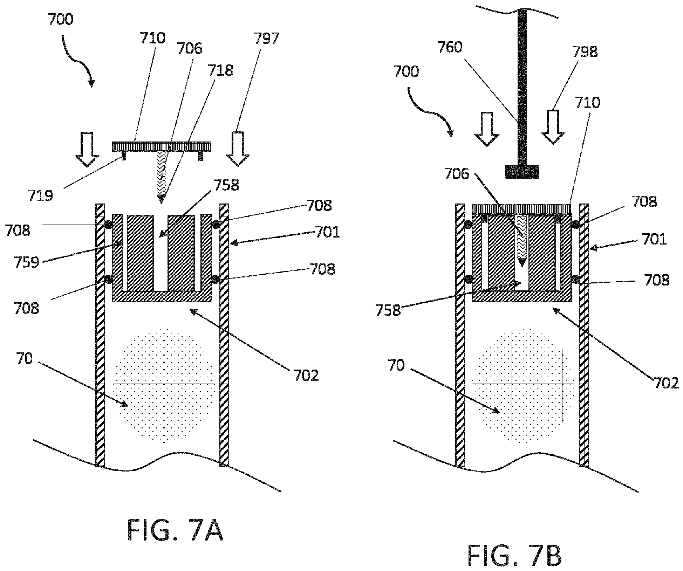

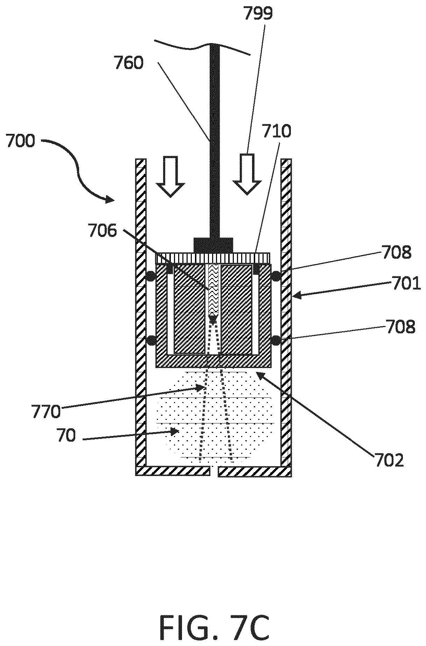

The stopper 400 also includes a cover 410 configured to interface with the substantially rigid shell 402 and accept a contact force from a head of a plunger (e.g., plunger rod 760 shown in FIGS. 7B, 7C) to advance the stopper 402 into the cartridge 114. The cover 410 may be constructed from a substantially rigid material, as detailed above with respect to the shell 402. The contact force from the plunger rod imparts a force to the cover 410 which is configured to transfer the force to the substantially rigid shell 402. The force transfer would be significantly reduced if the cover 410 is removed as the plunger may deform (e.g. by compressing) the insertable core 404. The cover 410 also provides a beneficial geometry for interfacing with the plunger 760 as it contacts a top surface of the rigid shell 402 along the circumference of the shell 402. Though a head of the plunger 760 may be sized to make contact with the top surface of the shell 402 (e.g. the diameter of the plunger 760 is larger than the diameter of the cavity of the shell), in some embodiments, including a cover 410 distributes the force more evenly on the shell 402.

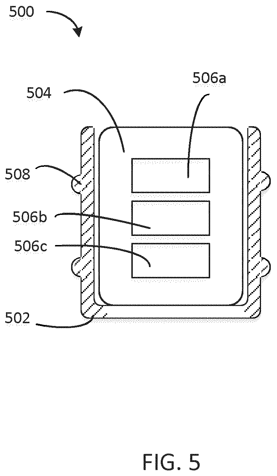

FIG. 5 is a cross-sectional view of a stopper configured to be disposed in an injection device, the stopper having a soft shell 502 and a rigid insertable core 504 with electronic devices embedded in the core 504. The stopper 500 includes a substantially soft shell 502 and a substantially rigid core 504. In this embodiment, the shell 502 and the core 504 are manufactured separately and the core 504 is inserted into the shell 502 in a subsequent assembly step. For example, the core 504 may be formed upon filling a mold with a substantially rigid material (e.g. by pouring, injecting, etc. the substantially rigid material) and embedded electronic devices 506a, 506b, and 506c before the substantially rigid material sets (e.g. cures, solidifies, etc.). In another embodiment, the substantially rigid material is introduced surrounding the electronic devices 506a, 506b, and 506c and shaped (e.g. cut, poured into a mold, etc.) to form the core 504. Integrated sealing element 508 is configured to provide a sealing interface with the cartridge 114 upon the stopper's introduction into the cartridge 114.

The soft shell 502 has the advantage that it has the shell function and sealing function in one component. However, in a manufacturing process it may be more difficult to handle a soft or elastomeric shell material for assembly. A rigid shell is easier to handle/hold during the manufacturing process and may lead to better dosing accuracy but requires separate sealing element like o-rings to provide the sealing interface to the cartridge. A substantially rigid core has the advantage of easier handling during manufacturing and better protection of possible stress sensitive sensor elements during heat sterilization.

The various materials from which the shell and the core could be produced as well as the various electronic devices that may be embedded in the core are described above with respect to FIG. 2A. Additionally, the material for the shell may be medical grade with the stopper being configured to come in contact with the medicament.

The cores 404 and 504 of the two-piece stoppers of FIG. 4 and FIG. 5 may be inserted after the shells 402 and 502 have been inserted into the cartridge 114. The shell and cartridge 114 may be heat sterilized. The cores 404 and 504, including their embedded electronics, may be pushed inside the cavity formed by the shell of the stopper after the cartridge 114 has been sterilized.

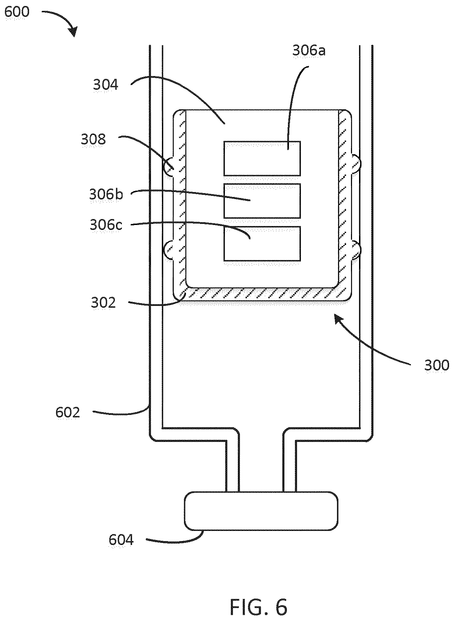

FIG. 6 is a cross-sectional view of a stopper disposed within a cartridge 600. The various features of the stopper shown are described above with respect to FIG. 3A and 3B. The stopper 300 may be replaced by any stopper of the previous figures, which would interface with the cartridge in much the same fashion as will be described here for stopper 300. The cartridge 600 includes a housing 602 which interfaces with the sealing element 308 of the stopper 300 to seal an open end of the cartridge 600. In some embodiments, a medicament (e.g., a medical fluid or drug) is disposed in the space between the cap 604 of the cartridge 600 and the shell 302 of the stopper 300. In some embodiments, the embedded electronics 306a, 306b, and 306c, include a sensor configured to send and receive a signal to sense a position of the stopper 300 in the cartridge 600.

The sensor may be a sensor/transceiver device such as, for example, a piezoelectric device. A sensor/transceiver may transmit a sensing signal, such as, for example, an ultrasonic, acoustic, light, or other signal through the stopper 300 and measure a response. The response received could be provided to a controller (e.g. an embedded or an external microcontroller) which may receive the response and calculate a state of the cartridge 600. The state of the cartridge 600 may correspond to, for example, a fill level of medicament in the cartridge 600 or a position of the stopper 300. The state of the cartridge may allow measurement of an injected dose of medicament. In some embodiments, different measuring methodologies are used to measure the position of the stopper 300 as described. A certain signal needs to be generated, which changes with the movement of the stopper 300 relative to a fixed position in the system or cartridge 600. This fixed position can be inside of the cartridge 600 e.g., the septum area of a cartridge 600 which never moves or another rigid wall of the cartridge 600, or an element which may be introduced into the cartridge for this purpose or the reference could be outside of the cartridge outside e.g. an element of the injection device like a housing part. In some embodiments, a sensor measures the change of a light signal with sending out the light from a light source (e.g., an LED) to the fixed area and receiving the remitted light with a photodetector. The intensity of the remission can be correlated to a distance. Another possibility is to measure the change of time needed for the signal (e.g. acoustic signal) to travel from a sender to the fixed position back to a receiver positioned close to the sender. In another embodiment the signal (optical, acoustic, capacitive etc.) can be sent out from a fixed position to a receiver in the moving stopper 300 to measure the change of the signal during stopper travel and correlate it to the stopper position in the cartridge 600.

FIG. 7A is cross-sectional view of a shell 702 of a stopper 700 disposed within a cartridge 701 and an insert 710 being coupled to the shell 702. The shell 702 is shown inside of the cartridge 701, with a plurality of sealing elements 708 surrounding the shell 702 and forming a seal to contain a medicament 70 in the cartridge 701. The stopper 702 has formed therein a central cavity 758 and peripheral alignment cavity 759. The insert 710 includes an alignment feature 719 extending from a bottom surface of the insert 710, along with an electronic device 706 coupled to the center of the insert 710 and arranged to be inserted into the central cavity 758 of the stopper 700 when the insert 710 is coupled with the shell 702 (as shown in FIG. 7B). In some embodiments, the shell 702 defines a receptacle for the insert 710. Additionally, the alignment feature 719 is arranged to be inserted into peripheral alignment cavity 759, and in some embodiments, includes a snap feature to secure the insert 710 to the shell 702, or, in some embodiments, is configured to be bonded to the insert 710 using an adhesive or weld. The insert 710 also includes a central alignment feature 718 on the electronic device 706 to aid in the positioning of the insert 710 during the initiation of an assembly operation, represented by arrows 797 indicating the direction of movement of the insert 710 (the result of which is shown in FIG. 7B). In some embodiments, during assembly, the guiding features (e.g., alignment feature 719 and central alignment feature 718) ensure the right orientation and accurate position of the insert 710 with respect to the shell 702.

The insert 710 and shell 702, in some embodiments, are constructed from of a rigid material such as metal, polymer (e.g., COC, PA, PP, PE, POM, PS, ABS, COP, etc.), glass or ceramics. In some embodiments, the electronic device (or electronic assembly) 706 includes one or more of the following: a sensor, a power source (e.g. battery), a controller, a wireless communication module (e.g. Bluetooth, NFC, Bluetooth LE, any RF, IrDA, Acoustic module, memory, on-off switch, thermo-sensing element, pressure sensor etc. In some embodiments, the sealing members 708 are made from a first material and the shell 702 is made from a second material of lower compressibility compared to the first member's material, and where the shell 702 is formed as a receptacle to house insert 710. In some embodiments, the electronic device 706 and insert 710 and are connected to the shell 702 using snap features, glue, welding, US welding, thermal welding or other means. In some embodiments, the insert 710 is removably coupled to the shell 702. In some embodiments, the sealing member 708 is made of a natural rubber or any biocompatible composition of rubber. In some embodiments, the insert 710 includes an on-off switch configured to trigger the electronics device 706 by any suitable impact on the stopper 700 (e.g. force from a plunger 760)

FIG. 7B is cross-sectional view the stopper 700 of FIG. 7A containing the insert 710 before being driven by a plunger 760 (which may itself be driven by the drive mechanism 106, as shown in FIG. 1) into the cartridge 701. FIG. 7B shows the insert 710 coupled to the shell 702, where the electronic device 706 is fully disposed within the central cavity 758 of the shell 702 and the alignment feature 719 is positioned within the peripheral alignment cavity 759. The plunger 760 (e.g., a plunger rod and head) is, in some embodiments, driven by an actuator or drive mechanism of an injector (as shown in FIG. 1) having the cartridge 701, or is a plunger of a syringe where the cartridge 701 is the syringe housing. In operation, the plunger 760 is driven (as indicated by arrows 798) against the insert 710 and thereby applies a force to the shell 702 to move the stopper 700 into the cartridge 701 in order to drive a portion of the medicament 70 from the cartridge 701.

FIG. 7C is cross-sectional view the stopper 700 of FIG. 7B during a sensing operation during or after being driven into the cartridge 701 by the plunger 760. FIG. 7C shows the plunger 760 contacting the stopper 700 and having driven the stopper 700 into the cartridge 701 (as shown by arrows 799). In operation, the electronics device 706 emits a sensing signal 770, which, in some embodiments, is responsive to the position of the stopper 700 in the cartridge 701 and enables the electronics device to generate a signal indication of the movement of the stopper 700.

Embodiments of the present disclosure can also apply to prefilled single and double chamber syringes that may not use a cartridge. The examples described above for integrating electronics into the stopper of a cartridge can also be used with disposable prefilled syringes. However, in the disposable syringe embodiments of FIGS. 8A and 8B, a threaded stopper-plunger rod interface is used to introduce an electronic device to the stopper by disposing the electronic device in the threaded end of the plunger rod and arranging a threaded cavity in the stopper to position the electronic device (when the plunger rod is threaded into the cavity) in a position to permit a sensing operation by the electronics device in the syringe to, for example, determine the position of the stopper in the syringe. For example, the threaded plunger is used to house specifically a sensor/connectivity technology, as shown in FIG 8A and 8B. This configuration enables the sensing features and connectivity features of the stopper detailed above to be used in disposable syringes. Additionally, in some embodiments, in a dual chamber syringe having a middle stopper and a tradition stopper configured to receive a plunger rod, aspects of the present disclosure may be integrated in the middle stopper during construction of the dual-chamber syringe in order to sense and/or measure the status of the device (e.g., "pre mixing", "mixed solution ready," or "injected completed" based on the sensing signal).

FIGS. 8A and 8B are cross-sectional views of a disposable syringe 800 having an electronic device 806 embedded into a threaded distal end 861 of a plunger 860. The stopper 802 includes threaded features 852 extending into a central cavity 859. The distal end 861 of the plunger 860 includes corresponded threaded features 862 arranged to secure the plunger 860 to the stopper 802. As detailed above, the stopper 802 is sealingly engaged to a cartridge, which is shown here as a housing 801 of the syringe, using a plurality of sealing members 808 disposed around the periphery of the stopper 802. In operation, the distal end 861 of the plunger 860 is moved (e.g., arrow 897) into contact with the stopper 802, and rotated (e.g., arrow 898) into threaded engagement with the stopper 802, as shown in FIG. 8B. In this configuration, the electronics device 806 is disposed in the stopper 802 and arranged to emit a sensing signal 870 in order to, in some embodiments, determine the position of the stopper 802 within the housing 801 of the syringe 800, or to determine if an injection operation has occurred. As shown in FIG. 8B, a force 899 is applied to the plunger 860 (e.g., by a patient or clinician operator of the syringe 800) and the plunger 860 drives the stopper 802 in the housing 801 of the syringe 800 in order to expel at least a portion of the medicament 80 from the syringe 800. One benefit of the disposable syringe 800 configuration of FIGS. 8A of 8B is the ability for the syringe housing 801 and stopper 802 to be subjected to heat sterilization prior to the introduction of the plunger 860 to the stopper 802, and thus eliminating a need to subject the electronic device 806 included in the distal end 861 of the plunger 860 to heat sterilization, which is a process that the electronic device 806 may be not able to functionally survive. Another advantage of this implementation is that the electronics device 806 is outside of the sterilization process. It can be manufactured more easily and cheaply in a typical manufacturing process. From a logistics perspective, introduction of the electronics device 806 may be done in a later manufacturing step and offers advantages regarding a longer battery life because the storage time can be optimized.

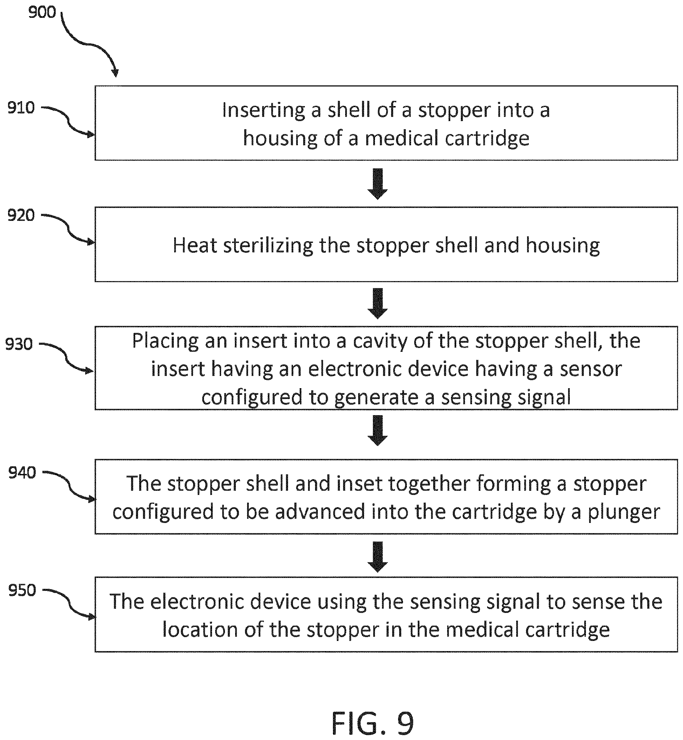

FIG. 9 is a flowchart depicting a method of assembling and sterilizing a cartridge with a stopper before coupling electronic elements into the stopper. First, a shell 702, 802 is inserted 910 in the cartridge 701, 801 of a medical cartridge 144, with the stopper 700, 802 sealing an open end of the cartridge 701, 801. Next, the assembled shell 702, 802 and cartridge 701, 801 are heat sterilized 920. Next, an insert 710, 861 having an electronic device 706, 806 is inserted 930 into a cavity 758, 859 of the housing, where the electronic device 706, 806 includes, in some embodiments, a sensor configured to generate a sensing signal 770, 870 responsive to the position of the shell 702, 802 in the cartridge 701, 801. The shell 702, 802 and insert 710, 861 being assembled forms 940 a stopper 700 able to be advanced by a plunger 760, 860 into the cartridge 701, 801. Finally, in some embodiments, the sensor of the electronic device 706, 806 uses the sensing signal to sense 950 the position of the stopper 700, 802 in the cartridge 701, 801 and, in some embodiments, wirelessly transmits a position signal to an electronic device external to the stopper 700, 802.

Described below are devices are methods for providing energy to electronic circuity in cartridge systems (for example, those disclosed herein) using energy harvesting to provide an alternative to standard batteries or as a supplement to batteries.

Aspects of the systems disclose above enable medical injectors to employ 'smart' technologies by way of an attached of embedded electronic component (e.g. RFID, sensor) to give a certain features to a cartridge of an injector device (e.g. of a pen-type injector). When integrating electronics into the stopper of a cartridge, a one or more components may be active (e.g., a sensor to measure certain properties of the injector or cartridge) and require an energy source, which typically could be a battery. One alternative, as described below, is to use a means of energy harvesting as a power source replacement for a battery.

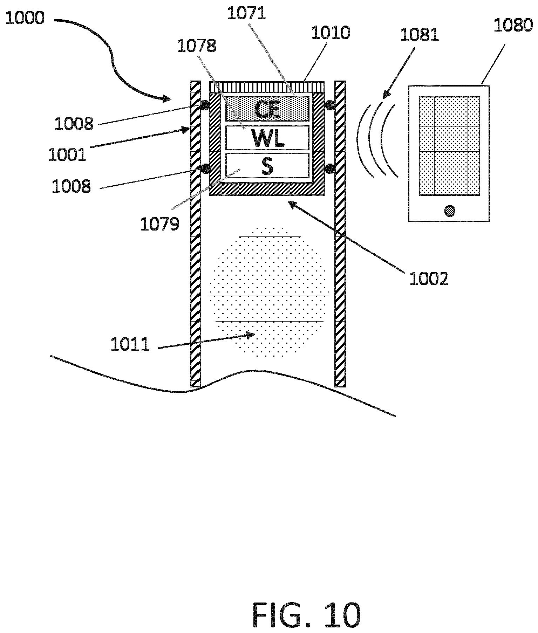

One example of an energy harvesting system in shown in FIG. 10, which is cross-sectional view of a stopper disposed within a cartridge and powered by a wireless system. FIG. 10 shows a cartridge 1000 including a stopper 1002 disposed in a housing 1001 of the cartridge 1000. The stopper 1002 and sealing elements 1008 together contain a medicament 1011 in the housing 1001 and the stopper 1002 includes a plurality of sealing elements 1008 disposed around the periphery of the stopper 1002 and sealingly engaged to an inner surface of the housing 1001. The stopper 1002 also includes a cap 1010 enclosing an opening of the stopper 1002, into which an electronics assembly 1071, 1078, 1079 has been inserted. In operation, the electronics assembly 1071, 1078, 1079 is, in some embodiments, integrated with or carried by the cap 1010, whereby securing the cap 1010 to the stopper 1002 includes inserting the electronics assembly 1071, 1078, 1079 into the cavity of the stopper 1002. The electronics assembly 1071, 1078, 1079 includes a sensing device (S) 1079, a wireless device (WL) 1078, and a capacitive device (CE) 1071.

In operation, the sensing device 1079 is configured to sense the position of the stopper 1002 within the housing 1001, and the wireless device 1078 is configured to communicate with an external electronic device (not shown) in order to communicate information from the sensor device 1079. The capacitive device 1071 is configured to provide electric power to the sensing device 1079 and the wireless device 1078 by way of wireless inductive charging from a wireless signal 1081 located in proximity to the cartridge 1000. In some embodiments, the capacitive device 1071 includes capacitive circuitry that is configured to receive power wirelessly from, for example, a smartphone 1080 via a nearfield communication protocol (NFC) signal 1081, or by a typical wireless charging device with other means of inductive loading, in order to provide enough energy for initiating and performing measurements with the sensing device 1079 in the cartridge 1000 and for transmitting back the results using the wireless device 1078.

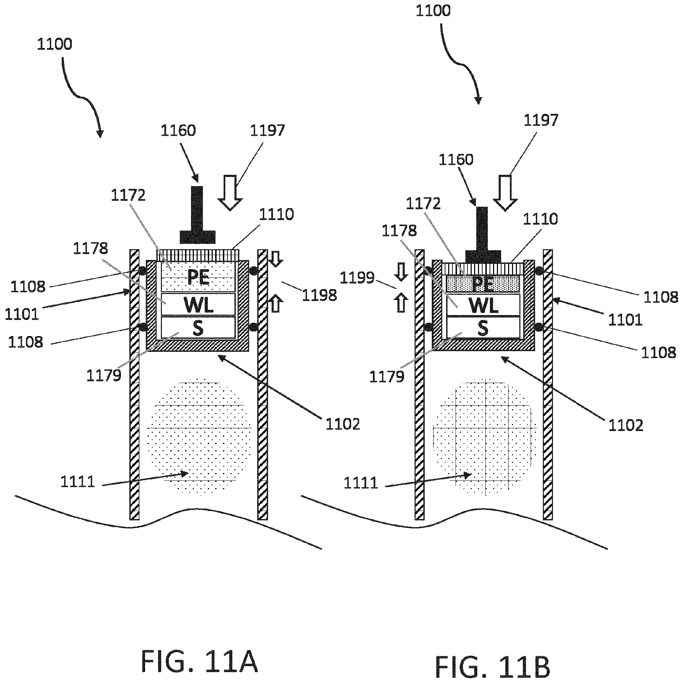

FIGS. 11A and 11B are cross-sectional views of a stopper disposed within a cartridge and powered by a piezoelectric system. Another example of an energy harvesting system is the use of a piezo technology to collect energy from the mechanical forces occurring in the between the stopper and plunger (or, for example the cartridge-stopper-threaded rod system of FIGS. 8A and 8B) during, for example, injector handling or an injection operation, to provide enough energy for initiating and performing the measurement in the cartridge and for transmitting back the results. FIG. 11A shows a cartridge 1100 having a stopper 1102 disposed in a housing 1101 of the cartridge 1100 and plunger 1160 advancing (e.g., along arrow 1197) to contact a cap 1110 of the stopper 1102. The stopper 1102 and sealing elements 1108 together contain a medicament 1111 in the housing 1101 and the stopper 1102 includes a plurality of sealing elements 1108 disposed around the periphery of the stopper 1102 and sealingly engaged to an inner surface of the housing 1101. The cap 1110 encloses an opening of the stopper 1102, into which an electronics assembly 1172, 1178, 1179 has been inserted, and the cap 1110 is configured to at least partially deflect under the force of the plunger 1160 or otherwise enable a transfer for force from the plunger to a portion of the electronics assembly 1172, 1178, 1179. In operation, the electronics assembly 1172, 1178, 1179 is, in some embodiments, integrated with or carried by the cap 1110, whereby securing the cap 1110 to the stopper 1102 includes inserting the electronics assembly 1172, 1178, 1179 into the cavity of the stopper 1102. The electronics assembly 1172, 1178, 1179 includes a sensing device (S) 1179, a wireless device (WL) 1178, and a piezoelectric element (PE) 1172.

In operation, the sensing device 1179 is configured to sense the position of the stopper 1102 within the housing 1101, and the wireless device 1178 is configured to communicate with an external electronic device (not shown) in order to communicate information from the sensor device 1179. The piezoelectric element 1172 is configured to provide electric power to the sensing device 1179 and the wireless device 1178 by way of transforming a portion of the force applied to the stopper 1102 into electric energy. As shown in FIG. 11B, the piezoelectric element 1172 is transformed from a position 1198 of FIG. 11A to a deflected position 1199 of FIG. 11B by the force applied to the cap 1110 by the plunger 1116 (during the motion indicated by arrow 1197). The transformation of the piezoelectric element 1172 from position 1198 to the deflected position 1199 absorbs energy and converts a portion of it to electric energy.

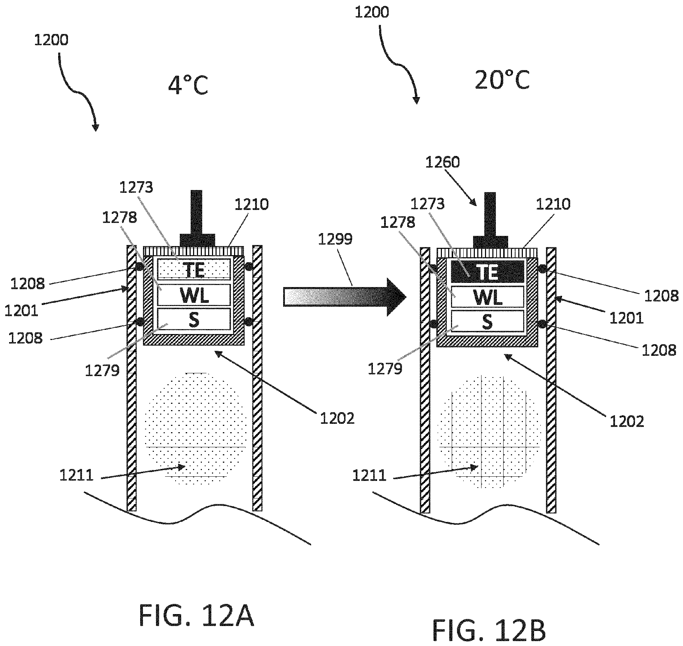

FIGS. 12A and 12B are cross-sectional views of a stopper disposed within a cartridge and including a Peltier thermoelectric device. Another example of an integrated energy harvesting device is the inclusion of thermoelectris elements (TE) to convert the temperature differences between refrigeration (e.g., of the pen or injector during storage) and warming (e.g., exposure to room temperatures) into electric energy to provide enough energy for initiating and performing the measurement in a cartridge of the injector/pen and for transmitting back the results. FIG. 12A shows a cartridge 1200 being stored in a low temperature environment (e.g., 4°C) and having a stopper 1202 disposed in a housing 1201 of the cartridge 1200 and plunger 1120 positioned against a cap 1210 of the stopper 1202. The stopper 1202 and sealing elements 1208 together contain a medicament 1211 in the housing 1201 and the stopper 1202 includes a plurality of sealing elements 1208 disposed around the periphery of the stopper 1202 and sealingly engaged to an inner surface of the housing 1201. The cap 1210 encloses an opening of the stopper 1202, into which an electronics assembly 1273, 1278, 1279 has been inserted, and the cap 1210 or stopper 1202 is configured to at least partially transfer thermal energy to the electronics assembly 1273, 1278, 1279. In operation, the electronics assembly 1273. 1278, 1279 is, in some embodiments, integrated with or carried by the cap 1210, whereby securing the cap 1210 to the stopper 1202 includes inserting the electronics assembly 1273, 1278, 1279 into the cavity of the stopper 1102. The electronics assembly 1273, 1178, 1179 includes a sensing device (S) 1279, a wireless device (WL) 1278, and a thermoelectric element (TE) 1273.

In operation, the sensing device 1279 is configured to sense the position of the stopper 1202 within the housing 1201, and the wireless device 1278 is configured to communicate with an external electronic device (not shown) in order to communicate information from the sensor device 1279. The thermoelectric element 1273 is configured to provide electric power to the sensing device 1279 and the wireless device 1278 by way of generating energy when the temperature of the thermoelectric element changes. As shown in FIG. 12B, the cartridge 1200 is moved to a relatively higher temperature environment (e.g., 20°C) and the thermoelectric element 1273 is heated by absorbing thermal energy from the environment outside the cartridge 1200 (during the temperature transition indicated by arrow 1299). The absorption of thermal energy by the thermoelectric element 1273 generates electric energy to power, for example, the sensing device 1279 and the wireless device 1278.

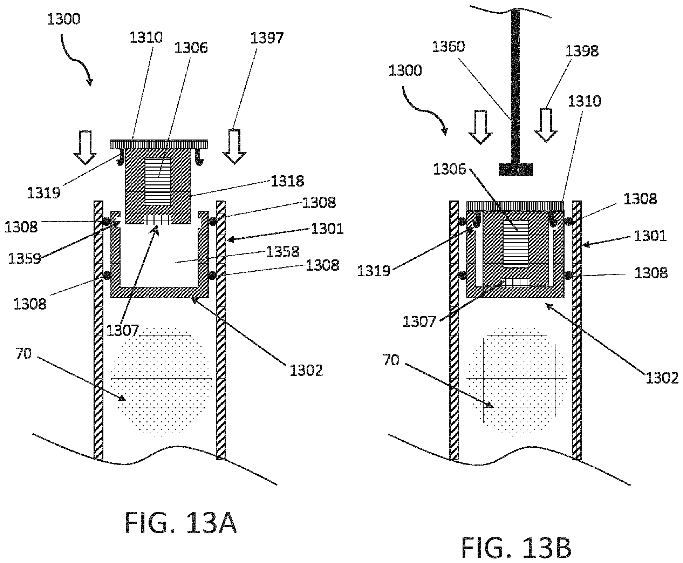

FIGS. 13A and 13B are cross-sectional views of a shell 1302 of a stopper 1300 disposed within a cartridge 1301 and an insert 1310 being clipped into a shell 1302. The shell 1302 is shown inside of the cartridge 1301, with a plurality of sealing elements 1308 surrounding the shell 1302 and forming a seal to contain a medicament 70 in the cartridge 1301. The stopper 1302 has formed therein a cavity 1358. The insert 1310 includes a snap feature 1319 extending from a bottom surface of the insert 1310, and a body 1318 with an electronic device 1306 in the center of the body 1318 and arranged to be inserted into the cavity 1358 of the stopper 1302 when the insert 1310 is coupled with the shell 1302 (as shown in FIG. 13B). The body 1318 of the insert 1310 includes a sensor 1307 disposed at the bottom of the insert in electrical communication with the electronic device 1306, and, in some embodiments, the sensor 1307 is arranged in the insert 1310 to send and receive a sensing signal though the shell 1302 and into the cartridge 1301. Additionally, the snap features 1319 are arranged to be inserted into side alignment cavities 1359 to secure the insert 1310 to the shell 1302, as shown in FIG. 13B. The body 1318 of the insert 1310 aids in the positioning of the insert 1310 during the initiation of an assembly operation, represented by arrows 1397 indicating the direction of movement of the insert 1310 (the result of which is shown in FIG. 13B).

The insert and shell 1302, in some embodiments, are constructed from of a rigid material such as metal, polymer (e.g., COC, PA, PP, PE, POM, PS, ABS, COP, etc.), glass or ceramics. In some embodiments, the electronic device (or electronic assembly) 1306 includes one or more of the following: a sensor, a power source (e.g. battery), a controller, a wireless communication module (e.g. Bluetooth, NFC, Bluetooth LE, any RF, IrDA, Acoustic module, memory, on-off switch, thermo-sensing element, pressure sensor, etc.). In some embodiments, the sealing members 1308 are made from a first material and the shell 1302 is made from a second material of lower compressibility compared to the first member's material, and where the shell 1302 is formed as a receptacle to house the insert 1310. In some embodiments, the insert 1310 is removably coupled to the shell 1302 after being snapped together. In some embodiments, the sealing member 1308 is made of a natural rubber or any biocompatible composition of rubber. In some embodiments, the insert 1310 includes an on-off switch configured to trigger the electronics device 1306 by any suitable impact on the stopper 1300 (e.g. force from a plunger 1360)

FIG. 13B is cross-sectional view the stopper 1300 of FIG. 13A containing the insert 1310 before being driven by a plunger 1360 into the cartridge 1301. FIG. 13B shows the insert 1310 coupled to the shell 1302, where the electronic device 1306 and sensor 1307 are fully disposed within the cavity 1358 of the shell 1302 and the snap features 1319 are positioned within the alignment cavities 1359. The plunger 1360 (e.g., a plunger rod and head) is, in some embodiments, driven by an actuator or drive mechanism of an injector (as shown in FIG. 1) having the cartridge 1301, or is a plunger of a syringe where the cartridge 1301 is the syringe housing. In operation, the plunger 1360 is driven (as indicated by arrows 1398) against the insert 1310 and thereby applies a force to the shell 1302 to move the stopper 1300 into the cartridge 1301 in order to drive a portion of the medicament 130 from the cartridge 1301.

Some of the features described can be implemented in digital electronic circuitry, or in computer hardware, firmware, software, or in combinations of them. The apparatus can be implemented in a computer program product tangibly embodied in an information carrier, e.g., in a machine-readable storage device, for execution by a programmable processor; and method steps can be performed by a programmable processor executing a program of instructions to perform functions of the described embodiments by operating on input data and generating output. The described features can be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a volume of a drug into a human or animal body. The volume can typically range from about 0.5 ml to about 10 ml. Without limitation, the drug delivery device may include a syringe, needle safety system, pen injector, auto injector, large-volume device (LVD), pump, perfusion system, or other device configured for subcutaneous, intramuscular, or intravascular delivery of the drug. Such devices often include a needle, wherein the needle can include a small gauge needle (e.g., greater than about 24 gauge, and including 27, 29, or 31 gauge).

In combination with a specific drug, the presently described devices may also be customized in order to operate within required parameters. For example, within a certain time period (e.g., about 3 to about 20 seconds for injectors, and about 5 minutes to about 60 minutes for an LVD), with a low or minimal level of discomfort, or within certain conditions related to human factors, shelf-life, expiry, biocompatibility, environmental considerations, etc. Such variations can arise due to various factors, such as, for example, a drug ranging in viscosity from about 3 cP to about 50 cP.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some embodiments, the chamber may be designed to store a drug for at least one day (e.g., 1 to at least 30 days). In some embodiments, the chamber may be designed to store a drug for about 1 month to about 2 years. Storage may occur at room temperature (e.g., about 20°C), or refrigerated temperatures (e.g., from about - 4°C to about 4°C). In some embodiments, the drug container may be or may include a dual-chamber cartridge configured to store two or more components of a drug formulation (e.g., a drug and a diluent, or two different types of drugs) separately, one in each chamber. In such embodiments, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyhepta¬decanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

A number of embodiments of the present disclosure have been described. The invention is defined by the appended claims.

### Reference numerals

- 70: medicament
- 100: injection device
- 104: cartridge housing
- 106: drive mechanism
- 108: stopper
- 109: needle
- 110: housing
- 111: injection button
- 112: dosage knob
- 113: dosage window
- 114: cartridge
- 115: needle assembly
- 116: inner needle cap
- 117: outer needle cap
- 118: cap
- 122: electronic component
- 124: electronic component
- 200: stopper
- 202: rigid shell
- 204: core
- 206a: electronic device
- 206b: electronic device
- 206c: electronic device
- 208: sealing element
- 300: stopper
- 302: soft shell
- 304: rigid core
- 306a: electronic device
- 306b: electronic device
- 306c: electronic device
- 308: sealing element
- 400: stopper
- 402: rigid shell
- 404: core
- 406a: embedded electronic device
- 406b: embedded electronic device
- 406c: embedded electronic device
- 408: sealing element
- 410: cover
- 500: stopper
- 502: soft shell
- 504: rigid insertable core
- 506a: embedded electronic device
- 506b: embedded electronic device
- 506c: embedded electronic device
- 508: sealing element
- 600: cartridge
- 602: cartridge housing
- 604: cap
- 700: stopper
- 701: cartridge
- 702: shell
- 706: electronic device
- 708: sealing elements
- 710: insert
- 718: central alignment feature
- 719: alignment feature
- 758: central cavity
- 759: peripheral alignment cavity
- 760: plunger
- 770: sensing signal
- 797: arrow
- 800: disposable syringe
- 801: housing
- 802: stopper
- 806: electronic device
- 808: sealing member
- 852: threaded features
- 859: central cavity
- 860: plunger
- 861: distal end
- 862: corresponded threaded features
- 870: sensing signal
- 897: arrow
- 898: arrow
- 899: force
- 910: inserted in the cartridge
- 920: heat sterilized
- 930: inserted into a cavity of the housing
- 940: shell and insert assembled into a stopper
- 950: sense the position of the stopper
- 1000: cartridge
- 1001: housing
- 1002: stopper
- 1008: sealing element
- 1010: cap
- 1011: medicament
- 1071: capacitive device (CE)
- 1078: wireless device (WL)
- 1079: sensing device (S)
- 1081: wireless signal
- 1080: smartphone
- 1100: cartridge
- 1101: housing
- 1102: stopper
- 1108: sealing element
- 1110: cap
- 1111: medicament
- 1160: plunger
- 1172: piezoelectric element (PE)
- 1178: wireless device (WL)
- 1179: sensing device (S)
- 1197: arrow
- 1200: cartridge
- 1201: housing
- 1202: stopper
- 1208: sealing element
- 1210: cap
- 1211: medicament
- 1260: plunger
- 1273: thermoelectric element (TE)
- 1278: wireless device (WL)
- 1279: sensing device (S)
- 1299: arrow
- 1300: cartridge
- 1301: housing
- 1302: stopper
- 1306: electronic device
- 1307: sensor
- 1308: sealing element
- 1310: cap
- 1311: medicament
- 1318: body
- 1319: snap feature
- 1359: side alignment cavity
- 1360: plunger
- 1397: arrow
- 1398: arrow

## Claims

1. A stopper (700, 1300) configured to be disposed within a medical cartridge (701, 1301), the stopper comprising:
a shell (702, 1302) defining a central cavity (758, 1318) and an exterior surface sized and shaped to fit inside the medical cartridge (701, 1301), the shell (202, 702, 1302) configured to be advanced into the medical cartridge (701, 1301) by a plunger (760, 1360) to apply pressure to a substance (70) contained in the medical cartridge(701, 1301) and
an insert (710, 1310) configured to be inserted into the cavity, the insert comprising an electronic device (706, 1306) having a sensor (1079, 1307) configured to generate a sensing signal, wherein the electronic device (706, 1306) is arranged to be inserted into the central cavity (758, 1318);
wherein the shell (702, 1302) is configured to pass the sensing signal (770) therethrough, and wherein the shell (702, 1302) defines a receptacle for the insert (710, 1310), and
wherein the sensor (1079, 1307) is configured to be responsive to the position of the stopper in the medical cartridge, **characterized in that**
one or more sealing elements ( 708, 1308) extending from the exterior surface of the shell (702, 1302), wherein the one or more sealing elements (708, 1308) are made from a first material and the shell (702, 1302) is made from a second material of lower compressibility compared to the first material,
wherein the shell (702, 1302) further defines a peripheral alignment cavity (759, 1359), wherein the insert (710, 1310) comprises an alignment feature (719, 1319) arranged to be inserted into the peripheral alignment cavity (759, 1359).

2. The stopper of claim 1, wherein the alignment feature (719, 1319) includes a snap feature to secure the insert (710, 1310) to the shell (702, 1302).

3. The stopper of claims 1 or 2, wherein the alignment feature (719, 1319) ensures a right orientation and accurate position of the insert (710, 1310) with respect to the shell (702, 1302).

4. The stopper of any of claims 1 to 3, wherein the sensing signal (770) is an electromagnetic signal or an acoustic signal.

5. The stopper of any of claims 1 to 4, wherein the electronic device (706, 1306) is configured to communicate with an external electronic device and wherein the electronic device (706, 1306) comprises a wireless transceiver (1078) configured to communicate with the external electronic device (1080).

6. The stopper of claim 5, wherein the wireless transceiver (1078) is configured to transmit data acquired by the sensor (1079, 1307) to the external electronic device (1080).

7. The stopper of any of claims 1 to 6, wherein the electronic device (706, 1306) is configured to be activated by a force (799, 899) applied to the stopper (108, 200, 700, 1300).

8. The stopper of any of claims 1 to 7, wherein the electronic device (706, 1306) comprises a temperature sensing element configured to activate the electronic device (706, 1306) upon sensing a change in temperature or upon reaching a temperature threshold.

9. The stopper of any of claims 1 to 8, wherein the electronic device (706, 1306) comprises an energy source (1071, 1172, 1273) and wherein the energy source comprises a wireless charging module having a capacitive electronic circuit (1071).

10. The stopper of claim 8, wherein the energy source comprises a piezoelectric element (1172) configured to generate electric energy from a force (799, 899) applied to the stopper (700, 1300).

11. The stopper of claim 8, wherein the energy source comprises a thermoelectric element (1273) configured to generate electric energy from heat energy absorbed by the stopper (700, 1300).

12. A medical cartridge (114, 600) comprising:
the cartridge housing (104, 602) and the stopper (700, 1300) of any of claims 1 to 11.

13. The medical cartridge (114, 600) according to claim 12, wherein the shell (702, 1302) of the stopper (700, 1300) is configured to be inserted into the cartridge housing (104, 602) prior to the cartridge housing (104, 602) and shell (702, 1302) undergoing a heat sterilization procedure, and the insert (710, 1310) of the stopper (700, 1300) is configured to be inserted into the stopper (700, 1300) after the heat sterilization procedure.

14. The medical cartridge according to claim 12 or 13 further containing a medicament.

15. A method of manufacturing the medical cartridge (114, 600) according to any one of the preceding claims 12 - 14, the method comprising:
inserting the shell (702, 1302) into the housing (104, 602) of the medical cartridge (701, 1302);
heat sterilizing the shell (702, 1302) and the housing (104, 602); and
after the heat sterilizing, placing the insert (710, 1310) into the central cavity (758, 1318) of the shell (702, 1302).

## Patentansprüche

1. Stopfen (700, 1300), der dazu konfiguriert ist, in einer medizinischen Kartusche (701, 1301) angeordnet zu werden, wobei der Stopfen Folgendes umfasst:
eine Schale (702, 1302), die einen zentralen Hohlraum (758, 1318) und eine Außenfläche, die so bemessen und geformt ist, dass sie in die medizinische Kartusche (701, 1301) passt, definiert, wobei die Schale (202, 702, 1302) dazu konfiguriert ist, in die medizinische Kartusche (701, 1301) durch einen Kolben (760, 1360) vorgeschoben zu werden, um Druck auf eine Substanz (70) auszuüben, die in der medizinischen Kartusche (701, 1301) enthalten ist, und
einen Einsatz (710, 1310), der dazu konfiguriert ist, in den Hohlraum eingesetzt zu werden, wobei der Einsatz eine elektronische Vorrichtung (706, 1306) mit einem Sensor (1079, 1307) umfasst, der dazu konfiguriert ist, ein Erfassungssignal zu erzeugen, wobei die elektronische Vorrichtung (706, 1306) angeordnet ist, um in den zentralen Hohlraum (758, 1318) eingesetzt zu werden;
wobei die Schale (702, 1302) dazu konfiguriert ist, das Erfassungssignal (770) durch diese zu leiten, und wobei die Schale (702, 1302) eine Aufnahme für den Einsatz (710, 1310) definiert, und
wobei der Sensor (1079, 1307) dazu konfiguriert ist, auf die Position des Stopfens in der medizinischen Kartusche zu reagieren, **dadurch gekennzeichnet, dass**
ein oder mehrere Dichtungselemente (708, 1308) sich von der Außenfläche der Schale (702, 1302) erstrecken, wobei das eine oder die mehreren Dichtungselemente (708, 1308) aus einem ersten Material hergestellt sind und die Schale (702, 1302) aus einem zweiten Material mit geringerer Kompressibilität im Vergleich zu dem ersten Material hergestellt ist,
wobei die Schale (702, 1302) ferner einen peripheren Ausrichtungshohlraum (759, 1359) definiert, wobei der Einsatz (710, 1310) ein Ausrichtungsmerkmal (719, 1319) umfasst, das angeordnet ist, um in den peripheren Ausrichtungshohlraum (759, 1359) eingesetzt zu werden.

2. Stopfen nach Anspruch 1, wobei das Ausrichtungsmerkmal (719, 1319) ein Schnappmerkmal beinhaltet, um den Einsatz (710, 1310) an der Schale (702, 1302) zu sichern.

3. Stopfen nach Ansprüchen 1 oder 2, wobei das Ausrichtungsmerkmal (719, 1319) eine richtige Ausrichtung und genaue Position des Einsatzes (710, 1310) in Bezug auf die Schale (702, 1302) gewährleistet.

4. Stopfen nach einem der Ansprüche 1 bis 3, wobei das Erfassungssignal (770) ein elektromagnetisches Signal oder ein akustisches Signal ist.

5. Stopfen nach einem der Ansprüche 1 bis 4, wobei die elektronische Vorrichtung (706, 1306) dazu konfiguriert ist, mit einer externen elektronischen Vorrichtung zu kommunizieren, und wobei die elektronische Vorrichtung (706, 1306) einen drahtlosen Sendeempfänger (1078) umfasst, der dazu konfiguriert ist, mit der externen elektronischen Vorrichtung (1080) zu kommunizieren.

6. Stopfen nach Anspruch 5, wobei der drahtlose Sendeempfänger (1078) dazu konfiguriert ist, durch den Sensor (1079, 1307) erfasste Daten an die externe elektronische Vorrichtung (1080) zu übertragen.

7. Stopfen nach einem der Ansprüche 1 bis 6, wobei die elektronische Vorrichtung (706, 1306) dazu konfiguriert ist, durch eine Kraft (799, 899) aktiviert zu werden, die auf den Stopfen (108, 200, 700, 1300) aufgebracht wird.

8. Stopfen nach einem der Ansprüche 1 bis 7, wobei die elektronische Vorrichtung (706, 1306) ein Temperaturerfassungselement umfasst, das dazu konfiguriert ist, die elektronische Vorrichtung (706, 1306) beim Erfassen einer Temperaturänderung oder bei Erreichen einer Temperaturschwelle zu aktivieren.

9. Stopfen nach einem der Ansprüche 1 bis 8, wobei die elektronische Vorrichtung (706, 1306) eine Energiequelle (1071, 1172, 1273) umfasst und wobei die Energiequelle ein drahtloses Lademodul mit einer kapazitiven elektronischen Schaltung (1071) umfasst.

10. Stopfen nach Anspruch 8, wobei die Energiequelle ein piezoelektrisches Element (1172) umfasst, das dazu konfiguriert ist, elektrische Energie aus einer Kraft (799, 899) zu erzeugen, die auf den Stopfen (700, 1300) aufgebracht wird.

11. Stopfen nach Anspruch 8, wobei die Energiequelle ein thermoelektrisches Element (1273) umfasst, das dazu konfiguriert ist, elektrische Energie aus Wärmeenergie zu erzeugen, die durch den Stopfen (700, 1300) absorbiert wird.

12. Medizinische Kartusche (114, 600), die Folgendes umfasst:
das Kartuschengehäuse (104, 602) und den Stopfen (700, 1300) nach einem der Ansprüche 1 bis 11.

13. Medizinische Kartusche (114, 600) nach Anspruch 12, wobei die Schale (702, 1302) des Stopfens (700, 1300) dazu konfiguriert ist, in das Kartuschengehäuse (104, 602) eingesetzt zu werden, bevor das Kartuschengehäuse (104, 602) und die Schale (702, 1302) einem Wärmesterilisationsvorgang unterzogen werden, und der Einsatz (710, 1310) des Stopfens (700, 1300) dazu konfiguriert ist, in den Stopfen (700, 1300) nach dem Wärmesterilisationsvorgang eingesetzt zu werden.

14. Medizinische Kartusche nach Anspruch 12 oder 13, die ferner ein Medikament enthält.

15. Verfahren zum Herstellen der medizinischen Kartusche (114, 600) nach einem der vorhergehenden Ansprüche 12 - 14, wobei das Verfahren Folgendes umfasst:
Einsetzen der Schale (702, 1302) in das Gehäuse (104, 602) der medizinischen Kartusche (701, 1302);
Wärmesterilisieren der Schale (702, 1302) und des Gehäuses (104, 602); und
nach dem Wärmesterilisieren, Platzieren des Einsatzes (710, 1310) in den zentralen Hohlraum (758, 1318) der Schale (702, 1302).

## Revendications

1. Bouchon (700, 1300) configuré pour être disposé dans une cartouche médicale (701, 1301), le bouchon comprenant :
une enveloppe (702, 1302) définissant une cavité centrale (758, 1318) et une surface extérieure dimensionnée et façonnée pour s'adapter à l'intérieur de la cartouche médicale (701, 1301), l'enveloppe (202, 702, 1302) configurée pour être avancée dans la cartouche médicale (701, 1301) par un piston (760, 1360) pour appliquer une pression à une substance (70) contenue dans la cartouche médicale (701, 1301) et
un insert (710, 1310) configuré pour être inséré dans la cavité, l'insert comprenant un dispositif électronique (706, 1306) ayant un capteur (1079, 1307) configuré pour générer un signal de détection, dans lequel le dispositif électronique (706, 1306) est agencé pour être inséré dans la cavité centrale (758, 1318) ;
dans lequel l'enveloppe (702, 1302) est configurée pour faire passer le signal de détection (770) à travers elle, et dans lequel l'enveloppe (702, 1302) définit un réceptacle pour l'insert (710, 1310), et
dans lequel le capteur (1079, 1307) est configuré pour être sensible à la position du bouchon dans la cartouche médicale, **caractérisé en ce que**
un ou plusieurs éléments d'étanchéité (708, 1308) s'étendant à partir de la surface extérieure de l'enveloppe (702, 1302), dans lequel le ou les éléments d'étanchéité (708, 1308) sont fabriqués à partir d'un premier matériau et l'enveloppe (702, 1302) est fabriquée à partir d'un second matériau de compressibilité inférieure à celle du premier matériau,
dans lequel l'enveloppe (702, 1302) définit en outre une cavité d'alignement périphérique (759, 1359), dans lequel l'insert (710, 1310) comprend un organe d'alignement (719, 1319) agencé pour être inséré dans la cavité d'alignement périphérique (759, 1359).

2. Bouchon selon la revendication 1, dans lequel l'organe d'alignement (719, 1319) comprend un organe cliquet pour fixer l'insert (710, 1310) à l'enveloppe (702, 1302).

3. Bouchon selon les revendications 1 ou 2, dans lequel l'organe d'alignement (719, 1319) assure une orientation correcte et une position précise de l'insert (710, 1310) par rapport à l'enveloppe (702, 1302).

4. Bouchon selon l'une quelconque des revendications 1 à 3, dans lequel le signal de détection (770) est un signal électromagnétique ou un signal acoustique.

5. Bouchon selon l'une quelconque des revendications 1 à 4, dans lequel le dispositif électronique (706, 1306) est configuré pour communiquer avec un dispositif électronique externe et dans lequel le dispositif électronique (706, 1306) comprend un émetteur-récepteur sans fil (1078) configuré pour communiquer avec le dispositif électronique externe (1080).

6. Bouchon selon la revendication 5, dans lequel l'émetteur-récepteur sans fil (1078) est configuré pour transmettre des données acquises par le capteur (1079, 1307) au dispositif électronique externe (1080).

7. Bouchon selon l'une quelconque des revendications 1 à 6, dans lequel le dispositif électronique (706, 1306) est configuré pour être activé par une force (799, 899) appliquée sur le bouchon (108, 200, 700, 1300).

8. Bouchon selon l'une quelconque des revendications 1 à 7, dans lequel le dispositif électronique (706, 1306) comprend un élément de détection de température configuré pour activer le dispositif électronique (706, 1306) lors de la détection d'un changement de température ou lors de l'atteinte d'un seuil de température.

9. Bouchon selon l'une quelconque des revendications 1 à 8, dans lequel le dispositif électronique (706, 1306) comprend une source d'énergie (1071, 1172, 1273) et dans lequel la source d'énergie comprend un module de charge sans fil ayant un circuit électronique capacitif (1071).

10. Bouchon selon la revendication 8, dans lequel la source d'énergie comprend un élément piézoélectrique (1172) configuré pour générer de l'énergie électrique à partir d'une force (799, 899) appliquée sur le bouchon (700, 1300).

11. Bouchon selon la revendication 8, dans lequel la source d'énergie comprend un élément thermoélectrique (1273) configuré pour générer de l'énergie électrique à partir de l'énergie thermique absorbée par le bouchon (700, 1300).

12. Cartouche médicale (114, 600) comprenant :
le boîtier de cartouche (104, 602) et le bouchon (700, 1300) selon l'une quelconque des revendications 1 à 11.

13. Cartouche médicale (114, 600) selon la revendication 12, dans laquelle l'enveloppe (702, 1302) du bouchon (700, 1300) est configurée pour être insérée dans le boîtier de cartouche (104, 602) avant le boîtier de cartouche (104, 602) et l'enveloppe (702, 1302) subissant une procédure de stérilisation thermique, et l'insert (710, 1310) du bouchon (700, 1300) est configuré pour être inséré dans le bouchon (700, 1300) après la procédure de stérilisation thermique.

14. Cartouche médicale selon la revendication 12 ou 13 contenant en outre un médicament.

15. Procédé de fabrication de la cartouche médicale (114, 600) selon l'une quelconque des revendications précédentes 12 à 14, le procédé comprenant :
l'insertion de l'enveloppe (702, 1302) dans le boîtier (104, 602) de la cartouche médicale (701, 1302) ;
la stérilisation thermique de l'enveloppe (702, 1302) et du boîtier (104, 602) ; et
après la stérilisation thermique, le placement de l'insert (710, 1310) dans la cavité centrale (758, 1318) de l'enveloppe (702, 1302).
